# EUROPEAN PATENT SPECIFICATION

(11) **EP 1 481 666 B1**
(45) Date of publication and mention of the grant of the patent: **13.10.2010**
(21) Application number: 04012486.9
(22) Date of filing: 26.05.2004
(51) Int. Cl.: A61K 9/00, A61K 31/19, A61K 31/715, A61P 15/02

(54) **A composition for re-establishment of the vaginal ecosystem**
Zusammensetzung zur Wiederherstellung des vaginalen Ökosystems
Composition pour le retablissement de l'écosystème vaginal

(30) Priority: 30.05.2003 IT TO20030404
(43) Date of publication of application: 01.12.2004
(73) Proprietor: ROTTAPHARM S.P.A., 20122 Milano (IT)
(72) Inventor: Senin, Paolo, 20052 Monza (IT); Setnikar, Ivo, 20146 Milano (IT); Tacconi, Elena, 27036 Mortara Pavia (IT); Rovati, Angelo Luigi, 20052 Monza (IT)
(74) Representative: Rambelli, Paolo

(56) References cited:
- EP-A- 0 257 007
- US-A1- 2003 180 366
- OLANO-MARTIN E ET AL: "IN VITRO FERMENTABILITY OF DEXTRAN, OLIGODEXTRAN AND MALTODEXTRIN BY HUMAN GUT BACTERIA" BRITISH JOURNAL OF NUTRITION, CAMBRIDGE, GB, vol. 83, no. 3, 2000, pages 247-255, XP001015480
- RYCROFT C E ET AL: "Fermentation properties of gentio-oligosaccharides" LETTERS IN APPLIED MICROBIOLOGY, vol. 32, no. 3, March 2001 (2001-03), pages 156-161, XP002293212 ISSN: 0266-8254

## Description

The present invention relates to a composition for vaginal use able to re-establish the physiological equilibrium of the vaginal ecosystem altered by an imbalance between Lactobacilli and other unwanted microbial species.

It is well known that the equilibrium of the vaginal ecosystem is regulated by the predominance of Lactobacilli which, by producing lactic acid, guarantee conditions of environmental acidity able to inhibit the increase of other unwanted micro-organisms the proliferation of which is inhibited by the acid environment.

When these conditions become altered for various reasons, such as inadequate intimate hygiene, prolonged menstrual flow, use of internal absorbents, contraceptive therapies and others, with consequent elevation of the vaginal pH, this natural self-defence mechanism is lost and the vaginal environment can be colonised by unwanted micro-organisms such as gram negative bacterial strains, then gardenella, anaerobes etc, with the appearance of troublesome symptoms such as itching, stinging and odorous discharges.

These symptoms, when they are not accompanied by an inflammatory state are identified with the term "vaginosis" and are not considered to be a real pathology. However they must be corrected and combated in a timely manner in order to avoid the onset of much more serious bacterial infections and of chronic inflammatory processes infecting the vagina.

The best way of re-establishing the equilibrium of the vaginal ecosystem appears therefore to be to recreate the optimum conditions for the reconstitution of a bacteria flora in which the Lactobacilli, as dominant species, by the production of lactic acid, regulate vaginal Ph with consequent inhibition of the growth of unwanted bacteria.

The reconstitution in the vagina of the physiological pH therefore starts off a chain of positive events which reinstate the conditions of the vaginal mucus to its natural and therefore optimal physiological state.

This positive feedback can be started by the introduction into the vagina of lactic acid to guarantee the reconstitution of the physiological pH, not greater than 4.5 and not less than 3.5. This condition inhibits the development of unwanted invasive microbial species, simultaneously permitting the Lactobacilli to become reinforced and to renew autonomously the production of lactic acid with the consequent maintenance of the physiological pH conditions.

In this way the need for further external interventions is eliminated, the vaginal environment being by now in a condition to manage itself in an optimum manner.

It is possible to improve the benefits of the lactic acid in the vaginal environment with other agents which synergise the action. These support factors are essentially represented by nutrient substrates for the Lactobacilli and by anti-microbial agents able selectively to eliminate the unwanted invasive species.

The presence of the above-mentioned agents, however, conceals risks which must be taken carefully into account, that is to say:
- the nutrient substrate, if not in correctly balanced quantities, can be utilised also by other micro-organisms such as, for example, fungi, candida, gram negatives etc, with consequent reversal from an advantageous situation to one which is transformed into an unwanted development of micro-organisms which damage the vaginal ecosystem;
- the anti-microbial agents, if not selected and quantitatively balanced, can inhibit not only the unwanted micro-organisms but also the Lactobacilli, in this way nullifying the positive effect of the lactic acid and making cyclic repetition of the treatment necessary. It is still to be added that the preservative systems have to be used with care, since some of these are irritant or allergenic and can therefore give rise to undesirable collateral effects.

EP-A-0 257 007 describes a composition for vaginal use having a pH lying between 3.5 and 4 including lactic acid and a nutrient substrate constituted by lactose or glycogen and free from preservatives.

Tests made by the applicant and reported in the present description have, however, detected that the use of glycogen as a nutrient substrate and the simultaneous absence of preservatives is not sufficient to inhibit the growth of unwanted micro-organisms, in particular *Aspergillus niger.*

### Detailed description of the invention

The object of the present invention is to provide a new composition for the reclamation and consequent normalisation of the vaginal ecosystem.

This object is achieved by a composition such as that defined in the following claims.

The main and optional constituents of the composition are hereinafter described, together with the rationale for their selection, and a description of their functions.

In the following description the percentages of individual components are percentages by weight except where indicated otherwise.
1. lactic acid and associated buffering agent (in the specific example preferably NaOH) in quantities such as to guarantee that the vaginal mucus has a pH lying between 3.5 and 4.5 and preferably 3.7-3.9. As already indicated the essential function of the lactic acid consists in creating a vaginal ecosystem favourable to the growth of Lactobacilli. This function is widely known and documented (see for example GB-A-253 218; GB-A-272 543; CA Vol. 105, 1986, Abstract No 49063y and EP 0 257 007) and therefore do not require further technical and scientific clarifications.
2. nutrient substrate able to encourage the growth of Lactobacilli without, at the same time, stimulating the growth of other, unwanted micro-organisms with the consequent risk of causing a deterioration in the state of the vaginal mucus, rather than achieving the primary objective of its normalisation.

To this end there exist numerous references in the scientific and Patent literature on the types of substrate tested and utilised, among which it is possible to cite lactose (see for example CA, Vol.105, 1986, Abstract no 49063y), glycogen (see EP 0 257 007), as well as the more easily bio-utilisable, that is to say glucose, all of which have been tested and rejected by the applicant on the basis of experimental evidence which will be described in more detail below.

In the present invention the nutrient substrate of choice comprises maltodextrins, never previously studied or tested for this type of application, which are surprisingly effective in supporting the growth of Lactobacilli in the presence of lactic acid and therefore in a suitably predisposed vaginal environment.

The experimental evidences, proving the utility of the use of maltodextrins, will be provided in the experimental part, from which it emerges that these are active if present in the composition in ratios by weight, in comparison with the lactic acid, lying between 0.04 and 0.4, preferably between 0.1 and 0.4; the preferred ratio being 0.2.
3. preservative system able to inhibit the growth of pathogenic and unwanted micro-organisms and at the same time not interfere with the other components of the composition in reconstituting and normalising the natural balance of the vaginal ecosystem.

This system preferably comprises, in the present invention, the association of two agents which synergise the respective preservative properties, which are free from allergenic effects and, above all, which do not interfere with the other components of the composition in stimulating the growth of Lactobacilli.

The two preferred substances are potassium sorbate and sodium methylparahydroxybenzoate, both well known and documented for their antibacterial and antifungal activity (see, for example, Handbook of Pharmaceutical Excipients - 3rd Edition - pages 431 and 340).

In this case also, for better security, the preservative system has been experimentally tested within the composition forming the subject of the present invention, confirming that its action is entirely secure and efficacious.
4. Formulative support constituted by inert excipients able to act as a vehicle for the assembly of the components active on the vaginal mucus, thereby guaranteeing a homogeneous distribution thereof and the contact time necessary to exert an effective normalising function regenerating the vaginal ecosystem itself.

The formulative support which is ideal for this type of application must be compatible with the active components and have a consistency and properties such as to perfom the first specified functions. The constituents which can form part of the formulative support comprise:
- semi-synthetic emulsifying and/or thickening agents (derived from variously substituted cellulose) such as hydroxypropyl cellulose (preferably in concentrations lying between 5 and 15%), hydroxypropylmethylcellulose (preferably in concentrations lying between 1 and 10%) and methylcellulose (preferably in concentrations lying between 2 and 4%);
- synthetic emulsifying and/or thickening agents such as polyacrylamide (preferably in concentrations lying between 2 and 6%), polyvinylalcohol (preferably in concentrations lying between 10 and 20%), alkyl ethers of polyoxyethylene (preferably in concentrations lying between 40 and 60%) and polyoxyamers (preferably in concentrations lying between 15 and 50%);
- surfactants such as cetosterearylic alcohol (preferably in concentrations lying between 6 and 10%);
- rubbers of various type (preferably in concentrations lying between 0.5 and 10%);
- proteins such as gelatine (preferably in concentrations lying between 2 and 5%);
- humectant and emollient agents such as glycerine in quantities not greater than 30% and/or propylene glycol in quantities preferably lying between 10 and 20%;
- water in quantities such as to bring the final percentage of the composition to 100 after having added together the relative percentages of all the other components.

### Experimental part

The experimental part has been undertaken for the purpose of achieving the objective of the present Patent Application and is fundamentally articulated in two tests, of which the first is dedicated to the identification of the possible nutrient substrate or substrates operable to encourage the optimum growth of Lactobacilli and the second to compare the efficacy of different formulations of the composition studied and formed according to the previously illustrated rationale.

### Test 1: comparison between nutrient substrates

Since the Lactobacilli control the vaginal pH by means of the production of lactic acid it appeared to be useful to verify, both directly and indirectly, their capacity to utilise the various substrates proposed for the production of lactic acid and consequently their utility in the formulation of the composition.

The test was structured in the following manner:
usable strains
   - *Lactobacillus acidophilus* ATCC 314 (human origin)
   - *Lactobacillus acidophilus wt* (from vaginal swab)
culture medium
   - MRS broth (Lactobacillus broth according to De Man, Rogose) Merck
   - MRS broth base (without D(+)-glucose)
   - Series of MRS broth base media to which maltodextrins were added in percentages lying between 0.1 and 5%
   - Series of MRS broth base media to which glycogen was added in quantities lying between 0.5 and 2%
   - Series of MRS broth base media to which glucose was added in quantities lying between 0.1 and 2%
   - Series of MRS broth base media to which lactose was added in quantities lying between 0.5 and 2%
**Note:** the pH of the media was always 5.7±0.2.

### Method

After having prepared fresh cultures of Lactobacilli in agar, an inoculation into five ml of MRS broth was incubated at 37°C for 24 hours.

Aliquotes were prepared from 60 ml of the previously described media and in each of these were inoculated 600 µl of the culture in agar, with subsequent incubation at 37°C for 24 hours.

As negative control aliquotes of 3 ml of the above-described media without inoculation of Lactobacilli were incubated. As positive control Merck MRS broth was utilised.

### Evaluation parameters

After 24 hours of incubation the optical density of the cultures was measured, the pH of the media was determined after centrifugation and filtration of the supernatant with a 0.45µ filter. The lactic acid present in the culture was also quantified by the HPLC method utilising as a control a laboratory standard of the same.

These evaluation parameters have the following significance:
- An increase in optical density confirms the growth of Lactobacilli;
- Reduction in pH indicates the formation of lactic acid;
- The quantitative determination of the lactic acid measures the efficiency of the use of the nutrient substrate by the Lactobacilli.

### Results

Table 1 plots the results obtained with the Lactobacillus ATCC 314. The results relating to *lactobacillus acidophilus wt* were the same and therefore have not been plotted.

**Table 1. Use of nutrient substrate by Lactobacillus ATCC 314**

| **Nutrient substrate** | **%** | **pH** | **D.O.** | **Lactic acid (%)** |
|---|---|---|---|---|
| | 0.5 | 5.95 | 0.148 | <0.10 |
| **GLY** | 1.0 | 5.93 | 0.213 | <0.10 |
| | 2.0 | 5.88 | 0.175 | <0.10 |
| | 0.5 | 5.88 | 0.218 | <0.10 |
| **LA** | 1.0 | 5.91 | 0.221 | <0.10 |
| | 2.0 | 5.94 | 0.179 | <0.10 |
| | 0.1 | 5.87 | 0.192 | <0.10 |
| | 0.5 | 5.73 | 0.486 | 0.24 |
| **MD** | 1.0 | 5.60 | 0.550 | 0.41 |
| | 2.0 | 5.34 | 0.958 | 0.62 |
| | 5.0 | 4.63 | 2.560 | 1.53 |
| **GLU** | 0.1 | 5.56 | 0.665 | 0.17 |
| | 0.5 | 4.74 | 1.540 | 0.90 |
| | 1.0 | 4.33 | 2.593 | 1.80 |
| | 2.0 | 4.57 | 2.540 | 2.43 |
| **MRS base** | | 5.89 | 0.126 | <0.10 |

| | | | | |
|---|---|---|---|---|
| Legend: GLY = Glycogen; LA = Lactose; MD = Maltodextrin; GLU = Glucose; MRS base = basic MRS broth without addition of D(+)-glucose and without inoculation of *Lactobacillus acidophilus* ATCC 314 (negative control). | | | | |

### Conclusions on the use of the various nutrient substrates

### - Glycogen and Lactose

The growth and production of lactic acid by *Lactobacillus acidophilus* ATCC 314 on these substrates, at concentrations from 0.5 to 2.0% was rather poor with respect to those observed with maltodextrin and glucose.

### - Maltodextrins

At a concentration of 0.1% they were not utilised for the production of lactic acid and, hardly, for the growth of Lactobacillus. At concentrations from 0.5 to 5.0%, on the other hand, they were utilised in a useful and dose-dependent manner both for the production of lactic acid and for the growth of Lactobacillus. They are therefore shown to be an optimum nutrient substrate for the composition.

### - Glucose

At a concentration of 0.1% it was hardly utilised for the production of lactic acid. On the other hand it was significant for the growth of Lactobacillus. At concentrations from 0.5 to 2.0% it was utilised in a useful and dose-dependent manner both for the production of lactic acid and for the growth of Lactobacillus.

In conclusion, maltodextrins and glucose are the nutrient substrates which present the most suitable characteristics for being utilised in the composition forming the subject of the present invention. Glucose, however, has limitations which will be illustrated and discussed below.

### Test 2: efficacy of various formulations of the composition

Various formulations of the composition forming the subject of the present invention were prepared and tested for the purpose of selecting which of them best responded to the rationale proposed for their practical use in normalisation of the vaginal ecosystem.

The test consisted in establishing the capacity of Lactobacilli and the other micro-organisms in competition with them to survive in the presence of an aliquote of the various formulations under test.

The following criteria were adopted:
- comparison between the growth of Lactobacilli and the other micro-organisms tested after inoculation in constant aliquotes of the various formulations under evaluation;
- comparative effect of the efficacy of the various nutrient substrates in encouraging or discouraging the growth of Lactobacilli with respect to the other micro-organisms in competition therewith;
- evaluation of the efficacy of the preservative system in blocking the growth of unwanted micro-organisms. The test "Efficacy of antimicrobial preservation" described in the Official Pharmacopea was utilised. The preservative system to be adopted must not however interfere with the growth of Lactobacilli.

In formulating the composition the following variables were taken into consideration:
- the concentration of the lactic acid;
- the type and concentration of the nutrient substrate; and
- the concentration and the type of inert excipients, that is to say humectants, thickening agents and aqueous vehicle.

The preservative system, where used, however, remained the same, constituted by potassium sorbate and sodium methylparahydroxybenzoate both in a concentration of 0.1%.

The pH was invariably fixed with the aid of a buffering agent (NaOH) at the physiological value existing in the vagina, that is to say at 3.8.

Some of the formulations tested are described in detail in the example part and are cited in the claims relating to the present invention.

The test was structured in the following manner.

### Tested strains

- Lactobacilli
   - *Lactobacillus acidophilus* ATCC 314
   - *Lactobacillus acidophilus* ATCC 4356
   - *Lactobacillus casei* subsp. *casei* ATCC 393
   - *Lactobacillus w.i.* (wild-type strain)
- Gram+, Gram- and potentially pathogenic fungi
   - *Pseudomonas aeroginosa* ATCC 9207
   - *Escherichia coli* ATCC 8739
   - *Candida albicans* ATCC 10231
   - *Staphylococcus aureus* ATCC 6538
   - *Aspergillus niger* ATCC 16404

These micro-organisms were subdivided into three groups identifiable thus:
- LB: all the strains of Lactobacilli;
- AM: the other micro-organisms tested with the exception of *Aspergillus niger;*
- AN: *Aspergillus niger*

### Culture media

- For the Lactobacilli:
   - MRS broth (Lactobacillus broth according to De Man, Rogosa and Sharpe)
   - MRS agar
- For the other micro-organisms
   - Tryptic Soy Agar
   - Tryptic Soy Broth
   - Sabouraud - 2% Glucose Agar (Merck).

### Method

The micro-organisms tested were inoculated in the respective liquid growth media and incubated for 24-48 hours until reaching a concentration of 10⁸-10⁹ cfu/ml.

After effecting titration of the cultures by seeding on a plate, 5g of the various formulations to be tested were inoculated with about 10⁶ cfu/ml of each strain.

### Evaluation parameters

The survival level of the micro-organisms under examination was evaluated by plating the suspension every 24 hours for five days and counting the cfu/ml.

To facilitate the interpretation, for each type of microorganism there was expressed an index of activity which summarises information both on the survival time and on the level of growth as follows:
- For Lactobacilli:
   (-): disappearance after 3 days
   (+) : presence of 10³-10⁴ cfu/ml after five days
   (++) :presence of 10⁴-10⁵ cfu/ml after five days .
- For the other micro-organisms, excluding *Aspergillus niger:*
   (-): disappearance of all the strains after two days
   (±): disappearance of all the strains except *Escherichia coli* and *Pseudomonas aeruginosa* after two days, the disappearance of which was observed on the test of the third day.
- For *Aspergillus niger:*
   (-): disappearance within three days
   (+): presence of - 10³ cfu/ml after five days
   (++): presence of - 10⁴ cfu/ml after five days
   (+++): undisturbed growth after five days

### Results

Table 2 plots the results obtained with the different formulations and lists them relating to:
- percentage of lactic acid;
- type and percentage of nutrient substrate (if present);
- presence or otherwise of the preservative system.

The percentage quantities and type of inert excipients were not plotted on the table since they did not influence the results of the tests.

The complete formulations are described in the example part.

All the strains of Lactobacilli (LB) tested gave results which can be superimposed and are indistinguishable and are therefore not individually reported.

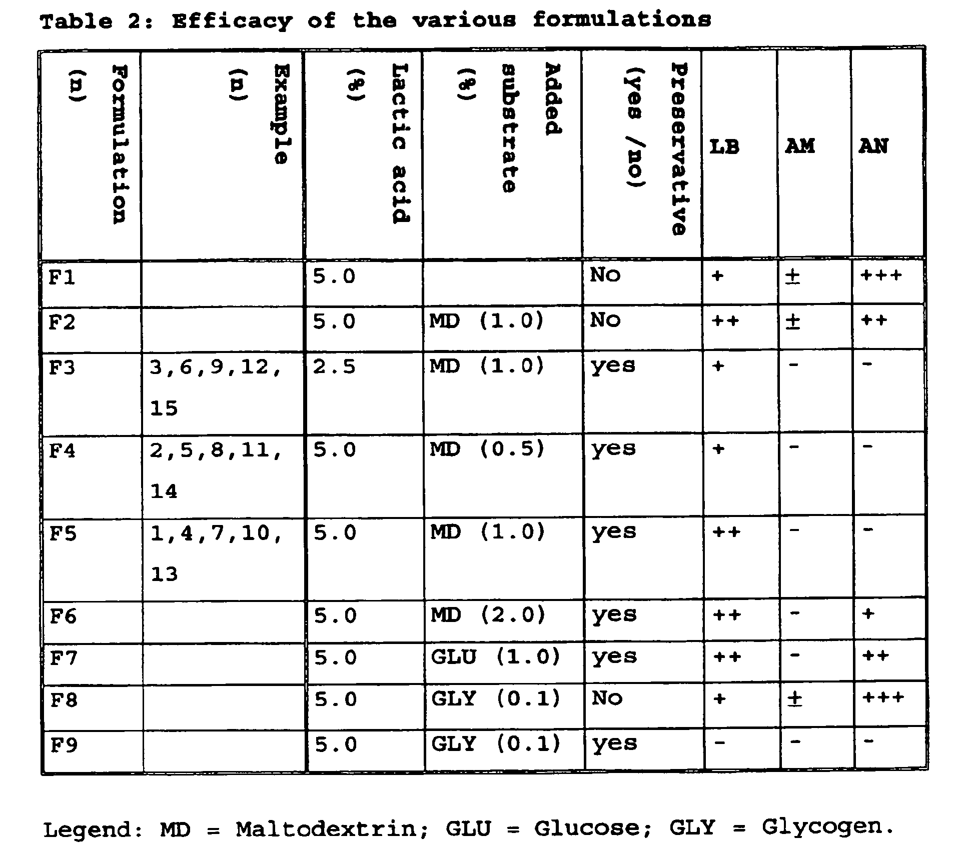

From the results of Table 2 it can be seen that the most advantageous formulations for the improvement in the vaginal ecosystem include the following components.
1. Lactic Acid , to regulate the pH to a physiological level. The optimum concentration was 5%, but also at half concentration, that is to say at 2.5%, its normalisation activity on the vaginal ecosystem was still evident (see F3). Consequently, the range of use of lactic acid in the formulation is preferably between 2.5 and 5%.
2. Maltodextrin, is an efficacious nutrient substrate. The maltodextrins were more efficacious than glycogen, as already indicated in Table 1.
   From the results relating to F3-F5 it emerges that:
   - the action of the maltodextrins at concentrations lying between 0.5 and 1% is sufficiently efficacious and selective since the Lactobacilli are able to utilise them usefully, whilst the other strains tested, and in particular *Aspergillus niger,* did not seem to be, also because of the concurrent effect of the preservative system;
   - from test number 2 it is moreover seen that the use of maltodextrins at concentrations greater than 1% were not useful (see F6) because the functional equilibrium between the lactic acid, nutrient substrate and preservative system was altered making it possible for an undesirable growth of *Aspergillus niger* to take place.
3. Glucose. Glucose is also an optimum nutrient substrate. It is not however usable because its action, highly stimulating the growth of the micro-organisms tested, was aspecific and uncontrollable inasmuch as in the case of F7, a simultaneous and unwanted development of *Aspergillus niger* was noted despite the presence of the preservative system. Therefore glucose has an uncontrollable effect and is not suitable for the proposed use.
4. Preservative system. Among the various potentially usable preservative systems was chosen, by way of example, that previously described based on potassium sorbate and sodium methylparahydroxybenzoate. This system, as demonstrated in the results of Table 2, blocks the possible growth of pathogenic or otherwise undesirable micro-organisms without at the same time inhibiting the growth of Lactobacilli, thereby satisfying the necessary and sufficient condition for its useful employment as preservative agent in the formulation forming the composition of the present invention.

Its essential function is further confirmed by the fact that in its absence (see F8, exact replicas of the formulation claimed in EP 0 257 007, and F1, which is the same without glycogen) the potentially pathogenic contaminants develop freely in the formulation without giving any advantageous increase in the growth of Lactobacilli.

Therefore a suitable preservative system is preferable for:
- impeding the microbial contamination of the formulations;
- inhibiting the growth of pathogenic strains in the vagina;
- validly reinforcing the action of the maltodextrins.

### Examples of formulation

The formulations indicated in the examples described hereinafter illustrate only some possible practical applications of the present invention and as such must not be considered limitative of the invention itself.

The method of preparation of the composition according to the illustrated examples does not require particular techniques or operative conditions, but only entirely routine and normal practical applications for those skilled in the specific art. The exemplary instructions for the performance of the process are hereinafter provided by way of illustration only.
- Introduce into a suitable turbo emulsifier the required quantities of purified water, lactic acid, sodium hydroxide, humectant and preservative system. Agitate until complete dissolution.
- Add under agitation the nutrient substrate and maintain under agitation until complete dissolution.
- Add, under agitation and at the required temperature, thickeners and emulsifiers to obtain an homogeneous distribution.
- Cool (if necessary) and eliminate incorporated air by agitation under vacuum.

The components of the formulations described in the examples described hereinafter are quantitatively expressed as percentages by weight.

The proportion to 100% is referred to purified water.

### Example No1:

- Lactic acid 5.0
- Maltodextrin 1.0
- Glycerine 15.0
- Hydroxypropylmethylcellulose 1.8
- Sodium methylparahydroxybenzoate 0.1
- Potassium sorbate 0.1
- Sodium hydroxide pH 3.8
- Water up to 100.0

### Example No 2:

As example No 1 with maltodextrin = 0.5%

### Example No 3:

As example No 1 with lactic acid = 2.5%

### Example No 4:

As example No 1 with propylene glycol (15%) in place of glycerine (15%)

### Example No 5:

As example No 2 with propylene glycol (15%) in place of glycerine (15%)

### Example No 6:

As example No 3 with propylene glycol (15%) in place of glycerine (15%)

### Example No 7:

As example No 1 with hydroxypropylcellulose (10%) in place of hydroxypropylmethyl cellulose (1.8%)

### Example No 8:

As example No 2 with hydroxypropylcellulose (10%) in place of hydroxypropylmethyl cellulose (1.8%)

### Example No 9:

As example No 3 with hydroxypropylcellulose (10%) in place of hydroxypropylmethyl cellulose (1.8%)

### Example No 10:

As example No 1 with methylcellulose (3%) in place of hydroxypropylmethyl cellulose (1.8%)

### Example No 11:

As example No 2 with methylcellulose (3%) in place of hydroxypropylmethyl cellulose (1.8%)

### Example No 12:

As example No 3 with methylcellulose (3%) in place of hydroxypropylmethyl cellulose (1.8%)

### Example No 13:

As example No 1 with polyvinylalcohol (15%) in place of hydroxypropylmethyl cellulose (1.8%)

### Example No 14:

As example No 2 with polyvinylalcohol (15%) in place of hydroxypropylmethyl cellulose (1.8%)

### Example No 15:

As example No 3 with polyvinylalcohol (15%) in place of hydroxypropylmethyl cellulose (1.8%)

## Claims

1. A composition suitable for application to the vagina for rebalancing the vaginal ecosystem by reconstitution of an environment which favours the development of Lactobacilli, comprising lactic acid for regulating the pH and a nutrient support for the Lactobacilli, **characterised in that** the said nutrient support comprises maltodextrin.

2. A composition according to Claim 1, **characterised in that** it further includes a preservative system suitable to protect the vaginal environment from possible unwanted pathogens or saprophytes.

3. A composition according to Claims 1 and 2, **characterised in that** the said preservative system comprises potassium sorbate and sodium methylparahydroxybenzoate.

4. A composition according to Claim 3, **characterised in that** it includes potassium sorbate and sodium methylparahydroxybenzoate in quantities of about 0.1% each by weight referred to the total weight of the composition.

5. A composition according to any preceding claim, **characterised in that** the lactic acid is contained in quantities lying between 2.5 and 5% by weight, preferably about 5% by weight.

6. A composition according to any preceding claim, **characterised in that** the maltodextrins are contained in quantities lying between 0.5 and 1% by weight, preferably about 1% by weight.

7. A composition according to any preceding claim, **characterised in that** it includes maltodextrins and lactic acid in a ratio by weight lying between 0.1 and 0.4 and preferably about 0.2.

8. A composition according to any preceding claim, including a buffering agent which does not interfere with the function of the composition, able to maintain the pH of the composition in the range from 3.5 to 4.5 and preferably from 3.7 to 3.9.

9. A composition according to Claim 8, in which the buffering agent is constituted by sodium hydroxide.

10. A composition according to any preceding claim, **characterised in that** it includes an aqueous vehicle and inert excipients chosen from the group consisting of thickening, emulsifying and humectant agents, and their mixtures.

11. A composition according to Claim 10, including glycerine as a humectant agent, in quantities not greater than 30% by weight and preferably about 15% by weight.

12. A composition according to Claim 10, **characterised in that** it includes propylene glycol as a humectant in quantities lying between 10 and 20% by weight and preferably about 15% by weight.

13. A composition according to Claim 10, **characterised in that** it includes hydroxypropylcellulose as a thickening agent in quantities between 5 and 15 % by weight and preferably about 10% by weight.

14. A composition according to Claim 10, **characterised in that** it includes hydroxypropylmethyl cellulose as thickening agent in quantities lying between 1 and 10% by weight and preferably about 5% by weight.

15. A composition according to Claim 10, **characterised in that** it includes methyl cellulose as a thickening agent in concentrations lying between 2 and 4% by weight and preferably about 3% by weight.

16. A composition according to Claim 10, **characterised in that** it includes polyacrylamide as a thickening agent in concentrations lying between 2 and 6% by weight and preferably about 4% by weight.

17. A composition according to Claim 10, **characterised in that** it includes polyvinylalcohol as a thickening agent in concentrations lying between 10 and 20% by weight, preferably about 15% by weight.

18. A composition according to Claim 10, **characterised in that** it includes alkyl ethers of polyoxyethylene as thickening agents in concentrations lying between 40 and 60% by weight and preferably about 30% by weight.

19. A composition according to Claim 10, **characterised in that** it includes polyoxamers as thickening agent in concentrations lying between 15 and 50% by weight, preferably about 30% by weight.

20. A composition according to Claim 10, **characterised in that** includes cetostearylic alcohol as thickening agent in concentrations lying between 6 and 10% by weight and preferably about 8% by weight.

21. A composition according to Claim 10, **characterised in that** it includes rubber of various types as thickening agent in concentrations lying between 0.5 and 10 % by weight and preferably about 5% by weight.

22. A composition according to Claim 10, **characterised in that** it includes protein, in particular gelatine, as thickening agent, in concentrations lying between 2 and 15% by weight and preferably about 8% by weight.

## Patentansprüche

1. Eine Zusammensetzung, welche zur Anwendung in der Vagina geeignet ist, um das vaginale Ökosystem wieder ins Gleichgewicht zu bringen, indem eine Umgebung wiederhergestellt wird, die die Entwicklung von Milchsäurebakterien begünstigt, umfassend Milchsäure zur Regulierung des pH-Wertes und einen Nährstoffträger für die Milchsäurebakterien, **dadurch gekennzeichnet, dass** der Nährstoffträger Maltodextrin umfasst.

2. Eine Zusammensetzung gemäß Anspruch 1, **dadurch gekennzeichnet, dass** sie weiterhin ein Schutzsystem einschließt, welches dafür geeignet ist, die vaginale Umgebung vor möglichen unerwünschten Pathogenen oder Saprophyten zu schützen.

3. Eine Zusammensetzung gemäß den Ansprüchen 1 und 2, **dadurch gekennzeichnet, dass** das Schutzsystem Kaliumsorbat und Natriummethylparahydroxybenzoat umfasst.

4. Eine Zusammensetzung gemäß Anspruch 3, **dadurch gekennzeichnet, dass** sie Kaliumsorbat und Natriummethylparahydroxybenzoat in Mengen von jeweils etwa 0.1 Gew.-%, bezogen auf das Gesamtgewicht der Zusammensetzung, einschließt.

5. Eine Zusammensetzung gemäß einem der vorstehenden Ansprüche, **dadurch gekennzeichnet, dass** die Milchsäure in Mengen, die zwischen 2,5 und 5 Gew.-% liegen, bevorzugt etwa 5 Gew.-%, enthalten ist.

6. Eine Zusammensetzung gemäß einem der vorstehenden Ansprüche, **dadurch gekennzeichnet, dass** die Maltodextrine in Mengen, die zwischen 0,5 und 1 Gew.-% liegen, bevorzugt etwa 1 Gew.-%, enthalten sind.

7. Eine Zusammensetzung gemäß einem der vorstehenden Ansprüche, **dadurch gekennzeichnet, dass** sie Maltodextrine und Milchsäure in einem Gewichtsverhältnis, das zwischen 0,1 und 0,4 liegt und bevorzugt etwa 0,2 ist, enthält.

8. Eine Zusammensetzung gemäß einem der vorstehenden Ansprüche, umfassend ein Puffermittel, welches die Funktion der Zusammensetzung nicht behindert, und welches dazu geeignet ist, den pH-Wert der Zusammensetzung im Bereich von 3,5 bis 4,5 und bevorzugt von 3,7 bis 3,9 zu halten.

9. Eine Zusammensetzung gemäß Anspruch 8, wobei das Puffermittel durch Natriumhydroxid dargestellt wird.

10. Eine Zusammensetzung gemäß einem der vorstehenden Ansprüche, **dadurch gekennzeichnet, dass** sie ein wässriges Vehikel und inerte Exzipienten, ausgewählt aus der Gruppe bestehend aus Verdickungsmitteln, Emulgierungsmitteln und Feuchthaltemitteln, und ihren Gemischen, einschließt.

11. Eine Zusammensetzung gemäß Anspruch 10, die Glycerin als Feuchthaltemittel in Mengen von nicht mehr als 30 Gew.-% und bevorzugt etwa 15 Gew.-% einschließt.

12. Eine Zusammensetzung gemäß Anspruch 10, **dadurch gekennzeichnet, dass** sie Propylenglykol als Feuchthaltemittel in Mengen, die zwischen 10 und 20 Gew.-% liegen, und welche bevorzugt etwa 15 Gew.-% ist, einschließt.

13. Eine Zusammensetzung gemäß Anspruch 10, **dadurch gekennzeichnet, dass** sie Hydroxypropylcellulose als Verdickungsmittel in Mengen zwischen 5 und 15 Gew.-%, und bevorzugt von etwa 10 Gew.-%, einschließt.

14. Eine Zusammensetzung gemäß Anspruch 10, **dadurch gekennzeichnet, dass** sie Hydroxypropylmethylcellulose als Verdickungsmittel in Mengen, die zwischen 1 und 10 Gew.-% liegen, und welche bevorzugt etwa 5 Gew.-% ist, einschließt.

15. Eine Zusammensetzung gemäß Anspruch 10, **dadurch gekennzeichnet, dass** sie Methylcellulose als Verdickungsmittel in Konzentrationen, die zwischen 2 und 4 Gew.-% liegen, und welche bevorzugt etwa 3 Gew.-% ist, einschließt.

16. Eine Zusammensetzung gemäß Anspruch 10, **dadurch gekennzeichnet, dass** sie Polyacrylamid als Verdickungsmittel in Konzentrationen, die zwischen 2 und 6 Gew.-% liegen, und welche bevorzugt etwa 4 Gew.-% ist, einschließt.

17. Eine Zusammensetzung gemäß Anspruch 10, **dadurch gekennzeichnet, dass** sie Polyvinylalkohol als Verdickungsmittel in Konzentrationen, die zwischen 10 und 20 Gew.-% liegen, bevorzugt etwa 15 Gew.-%, einschließt.

18. Eine Zusammensetzung gemäß Anspruch 10, **dadurch gekennzeichnet, dass** sie Alkylether von Polyoxyethylen als Verdickungsmittel in Konzentrationen, die zwischen 40 und 60 Gew.-% liegen, und welche bevorzugt etwa 30 Gew.-% ist, einschließt.

19. Eine Zusammensetzung gemäß Anspruch 10, **dadurch gekennzeichnet, dass** sie Polyoxamere als Verdickungsmittel in Konzentrationen, die zwischen 15 und 50 Gew.-% liegen, bevorzugt etwa 30 Gew.-%, einschließt.

20. Eine Zusammensetzung gemäß Anspruch 10, **dadurch gekennzeichnet, dass** sie Cetostearyl-Alkohol als Verdickungsmittel in Konzentrationen, die zwischen 6 und 10 Gew.-% liegen, und welche bevorzugt etwa 8 Gew.-% ist, einschließt.

21. Eine Zusammensetzung gemäß Anspruch 10, **dadurch gekennzeichnet, dass** sie Kautschuk von verschiedenen Arten als Verdickungsmittel in Konzentrationen, die zwischen 0,5 und 10 Gew.-% liegen, und welche bevorzugt etwa 5 Gew.-% ist, einschließt.

22. Eine Zusammensetzung gemäß Anspruch 10, **dadurch gekennzeichnet, dass** sie Eiweiß, insbesondere Gelatine, als Verdickungsmittel in Konzentrationen, die zwischen 2 und 15 Gew.-% liegen, und welche bevorzugt etwa 8 Gew.-% ist, einschließt.

## Revendications

1. Composition convenant à l'application vaginale pour rééquilibrer l'écosystème vaginal par reconstitution d'un environnement favorisant le développement des Lactobacillus, comprenant de l'acide lactique destiné à réguler le pH et un support nutritionnel pour les Lactobacillus, **caractérisée en ce que** ledit support nutritionnel comprend de la maltodextrine.

2. Composition selon la revendication 1, **caractérisée en ce qu'**elle comprend en outre un système de conservation apte à protéger l'environnement vaginal d'éventuels agents pathogènes ou saprophytes indésirables.

3. Composition selon les revendications 1 et 2, **caractérisée en ce que** ledit système de conservation comprend du sorbate de potassium et du méthylparahydroxybenzoate de sodium.

4. Composition selon la revendication 3, **caractérisée en ce qu'**elle comprend du sorbate de potassium et du méthylpara-hydroxybenzoate de sodium en des quantités respectives d'environ 0,1 % en poids par rapport au poids total de la composition.

5. Composition selon l'une quelconque des revendications précédentes, **caractérisée en ce que** l'acide lactique est contenu en des quantités comprises entre 2,5 et 5 % en poids, de préférence égales à environ 5 % en poids.

6. Composition selon l'une quelconque des revendications précédentes, **caractérisée en ce que** les maltodextrines sont contenues en des quantités comprises entre 0,5 et 1 % en poids, de préférence égales à environ 1 % en poids.

7. Composition selon l'une quelconque des revendications précédentes, **caractérisée en ce qu'**elle comprend des maltodextrines et de l'acide lactique en un rapport en poids compris entre 0,1 et 0,4 et de préférence égal à environ 0,2.

8. Composition selon l'une quelconque des revendications précédentes, incluant un agent de tamponnement qui n'interfère pas avec la fonction de la composition, apte à maintenir le pH de la composition dans la plage de 3,5 à 4,5 et de préférence de 3,7 à 3,9.

9. Composition selon la revendication 8, dans laquelle l'agent de tamponnement est constitué par de l'hydroxyde de sodium.

10. Composition selon l'une quelconque des revendications précédentes, **caractérisée en ce qu'**elle comprend un véhicule aqueux et des excipients inertes choisis dans le groupe constitué d'agents épaississants, émulsifiants et humidifiants et leurs mélanges.

11. Composition selon la revendication 10, comprenant du glycérol comme agent humidifiant, en des quantités inférieures ou égales à 30 % en poids et de préférence égales à environ 15 % en poids.

12. Composition selon la revendication 10, **caractérisée en ce qu'**elle comprend du propylèneglycol comme agent humidifiant en des quantités comprises entre 10 et 20 % en poids et de préférence égales à environ 15 % en poids.

13. Composition selon la revendication 10, **caractérisée en ce qu'**elle comprend de l'hydroxypropylcellulose comme agent épaississant en des quantités comprises entre 5 et 15 % en poids et de préférence égales à environ 10 % en poids.

14. Composition selon la revendication 10, **caractérisée en ce qu'**elle comprend de l'hydroxypropylméthylcellulose comme agent épaississant en des quantités comprises entre 1 et 10 % en poids et de préférence égales à environ 5 % en poids.

15. Composition selon la revendication 10, **caractérisée en ce qu'**elle comprend de la méthylcellulose comme agent épaississant en des concentrations comprises entre 2 et 4 % en poids et de préférence égales à environ 3 % en poids.

16. Composition selon la revendication 10, **caractérisée en ce qu'**elle comprend du polyacrylamide comme agent épaississant en des concentrations comprises entre 2 et 6 % en poids et de préférence égales à environ 4 % en poids.

17. Composition selon la revendication 10, **caractérisée en ce qu'**elle comprend du poly(alcool vinylique) comme agent épaississant en concentrations comprises entre 10 et 20 % en poids, de préférence égales à environ 15 % en poids.

18. Composition selon la revendication 10, **caractérisée en ce qu'**elle comprend des éthers alkyliques de polyoxyéthylène comme agents épaississants en des concentrations comprises entre 40 et 60 % en poids et de préférence égales à environ 30 % en poids.

19. Composition selon la revendication 10, **caractérisée en ce qu'**elle comprend des polyoxamères comme agents épaississants en des concentrations comprises entre 15 et 50 % en poids, de préférence égales à environ 30 % en poids.

20. Composition selon la revendication 10, **caractérisée en ce qu'**elle comprend de l'alcool cétostéarylique comme agent épaississant en des concentrations comprises entre 6 et 10 % en poids et de préférence égales à environ 8 % en poids.

21. Composition selon la revendication 10, **caractérisée en ce qu'**elle comprend du caoutchouc de divers types comme agent épaississant en des concentrations comprises entre 0,5 et 10 % en poids et de préférence égales à environ 5 % en poids.

22. Composition selon la revendication 10, **caractérisée en ce qu'**elle comprend une protéine, en particulier de la gélatine, comme agent épaississant en des concentrations comprises entre 2 et 15 % en poids et de préférence égales à environ 8 % en poids.
